(19) Europäisches Patentamt
European Patent Office
Office européen des brevets

(11) Publication number: 0 006 752
B1

(12) EUROPEAN PATENT SPECIFICATION

(45) Date of publication of patent specification: 22.12.82

(21) Application number: 79301233.7

(22) Date of filing: 26.06.79

(51) Int. Cl.³: A 61 K 31/415,
C 07 D 413/04,
C 07 C 107/06,
A 61 K 31/655

# (54) Compositions for the treatment of chronic trypanosomiasis infections.

(30) Priority: 28.06.78 GB 2815078

(43) Date of publication of application:
09.01.80 Bulletin 80/1

(45) Publication of the grant of the patent:
22.12.82 Bulletin 82/51

(84) Designated Contracting States:
BE CH DE FR IT LU NL SE

(56) References cited:
US - A - 4 010 176
Römpps Chemie-Lexikon, Vol. 1, p. 345 (1972)

(73) Proprietor: MERCK & CO. INC.
126, East Lincoln Avenue P.O. Box 2000
Rahway, New Jersey 07065 (US)

(72) Inventor: Jennings, Francis William
7 Fifth Avenue
Glasgow G12 OAS (GB)

(74) Representative: Crampton, Keith John Allen et al,
D YOUNG & CO 10 Staple Inn
London WC1V 7RD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 006 752 B1

Courier Press, Leamington Spa, England.

# Compositions and methods for the treatment of chronic trypanosomiasis infections

## BACKGROUND OF THE INVENTION

Trypanosomiasis is a term used to describe a group of allied protozoal diseases, each of which is due to infection with a species of the genus *Trypanosoma.* They reach great importance in Africa where their presence in enzootic form precludes the keeping of domestic animals throughout the largest part of the continent between 15°N and 20°S latitude. The pathogenic trypanosomes of Africa are considered to be primarily associated with the tsetse flies (glossina) which feed on vertebrate blood. The tsetse is the vector and also is the host in which part of the trypanosome life cycle takes part. Wherever tsetse are present, trypanosomiasis will also be found in some part of the mammalian population. The clinical findings are typically those of a wasting disease with intermittent fever. Anemia, edema, and cachexia are parts of the syndrome.

The important trypanosomes pathogenic to domestic animals are *T. congolense, T. vivax, T. simiae, T. suis, T. equiperdum,* and *T. brucei.* The latter trypanosome is morphologically identical to *T. gambiense,* and *T. rhodesiense,* responsible for human "sleeping sickness" of Africa. The tsetse fly is the vector in the transmittal of these trypanosomes. The trypanosomes *T. evansi* and *T. vivax* are also mechanically transmitted by bloodfeeding insects. One trypanosome found in the Western Hemisphere is *T. cruzi,* which infects both animals and man and in man causes a very serious condition known as Chagas' disease.

Acute Chagas' disease *(T. cruzi)* occurs in people of all ages but is especially important in the adolescent. The chronic form may be mild and asymptomatic, although it frequently gives rise to myocarditis and N.S. (nervous system) involvement resulting in morbidity and premature mortality.

A recurring problem in the chemotherapy of trypanosomiasis is the patient or animal which relapses after treatment. If the drug fails to clear the circulating parasitaemia or if the parasites reappear in the bloodstream after an aparasitaemic phase which varies depending on the persistence and level of the drugs used, then there is a distinct possibility that either underdosage of the drug is the causitive factor or drug resistance has occurred. If, however, there is a prolonged aparasitaemic phase after chemotherapy before the eventual reappearance of the parasite this, in many instances, has been attributed to reinfection if the animal has remained in the endemic area. The other possibility is the existence of an "occult" phase of the disease, which gives rise to a new cycle of parasitaemia.

This was described in greater detail in the case of *T. brucei* TREU 667/1 infection in the mouse by Jennings, Whitelaw & Urquhart, *Parasitol.* 75, 143—153 (1977), who found that infections of mice with this stabilate could be cured permanently if treatment was initiated early in the infection (3—7 days), but that if it were delayed (14—21 days), then nearly all the mice eventually relapsed despite the mice being treated with many times the recommended dose of drugs.

The present invention provides a composition diminazene aceturate and nitroimidazole having the structure

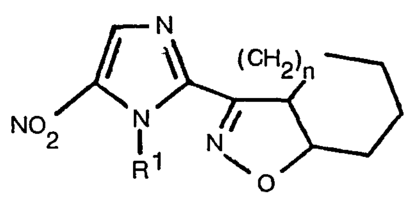

wherein $R^1$ is hydrogen or $C_{1-6}$ alkyl, and *n* is 1 or 3 in a weight ratio of 1—8:2—32.

It should be noted that nitroimidazoles having trypanosomal activity are described in United States Patents Nos. 3,711,495 (which discloses certain specific nitroimidazoles used in accordance with the present invention, as well as a class of nitroimidazoles that includes those used in accordance with the present invention), 3,915,978, 4,010,176, 3,646,027 and 3,737,547.

The following non-limiting Examples will serve to further illustrate the invention.

## MATERIALS AND ASSAY METHODS

The mice used were adult CFLP females approximately 25—30 g. and maintained on standard laboratory diet throughout the experiments.

Two stabilates were used throughout the experiments, the main one was *T. brucei* TREU 667/1 originally obtained from Dr A R Gray, Edinburgh and the other *T. brucei* LUMP 1001 obtained from Professor W H R Lumsden, London. These were cryogenically maintained as previously described in Jennings, Whitelaw & Urquhart, *Parasitol. 75,* 143—153 (1977).

Each mouse was inoculated intraperitoneally with 1 × $10^4$ trypanosomes prepared from the stabilate in phosphate glucose saline pH 8.0. Chemotherapy commenced at various times post inoculation. Each mouse was weighed and the requisite dose of drug administered intraperitoneally in a volume of 0.1 ml./5g. body weight.

The nitroimidazoles tested were:

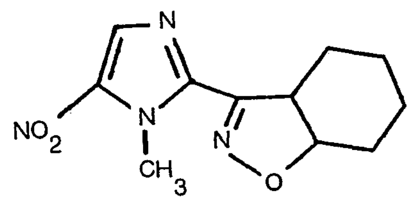

I. 3′-(1-methyl-5-nitroimidazol-2-yl)-4′5′-tetramethylene-$\Delta^2$-isoxazoline

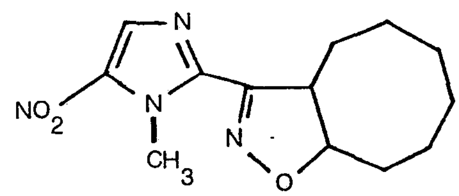

II. 3′-(1-methyl-5-nitroimidazol-2-yl)-4′,5′-hexamethylene-$\Delta^2$-isoxazoline

Hereinafter, the above named Compounds are referred to by their designated Roman numerals.

Compounds I, and II were used in crystalline powder forms. These were first ground finely in an agate mortar and then suspended in physiological saline for intraperitoneal injection. A drop of detergent (Tween 80) was added to the suspensions to facilitate wetting. Care was taken during dispensing that the suspensions were properly dispersed, and because of some slight unavoidable settlement in the syringe, the mice were always injected at random and usually not more than 3 mice were injected from the one syringe full of drug suspension. As the regimen was usually 4 daily doses at the respective drug concentration, any errors were evenly distributed.

The other established drugs used were those sold under the trade mark Berenil (diminazene aceturate, obtained from Farbwerke, Hoechst, Germany, 40 mg./kg. unless otherwise specified), Ethidium (homidium bromide 7.5 mg./kg.) and Prothidium (pyrithidium bromide 10 mg./kg. obtained from Boots Pure Drug Co., England) and Samorin (isometamidium 2 mg./kg. obtained from May & Baker Ltd.). All were prepared according to the manufacturer's instructions and were injected intraperitoneally.

The mice were examined for parasites on the day treatment was initiated (usually day 3 or 21 of infection) by examination of a drop of tail blood under ×40 objective (wet film technique). Subsequent to treatment they were examined every 2 days for 6 days and thereafter once per week until relapse occurred or until the experiment was terminated (in some instances 6—7 months).

The mice which showed relapse parasitaemias were removed from their experimental groups to a separate holding cage after they showed a positive wet blood film on two separate occasions.

The following Examples contain data that illustrate the invention as well as comparative data showing the smaller effects of the two ingredients on their own and of other combinations of ingredients.

Example 1

The Effect of Compounds I and II on Early Infections of *T. brucei* TREU 667/1

The two compounds I and II were used at various dose levels against an early infection of *T. brucei* TREU 667/1 in CFLP. The dose regimen was initiated 3 days after infection and the intraperitoneal injections of drug suspension were given daily. The results are set forth in Table 1:

TABLE 1

Effectiveness of Compounds I and II When Administered to Mice Infected For 3 Days With *Trypanosoma brucei* TREU 667/1

| Drug | Number and Level of Daily Doses | Number Cured* Out of Total Treated |
|---|---|---|
| I | 4 x 5 mg./kg. | 0/8 |
| | 4 x 20 mg./kg. | 2/8 |
| I** | 4 x 80 mg./kg. | 10/10 |
| II | 4 x 5 mg./kg. | 2/8 |
| | 4 x 20 mg./kg. | 8/8 |

* Cure indicates that relapses did not occur, i.e., that there was a complete absence of parasites in the blood for at least 90 days after treatment or till the end of the experiment.

** Separate experiment.

The initial results (Table 1) using dose regimens of 4 daily doses at 5 and 20 mg./kg. of Compound I did not result in any cures. Compound II, 4 doses at 20 mg./kg. gave 100% cure of a 3-day old infection. There was some indication of activity with Compound II administered in 4 doses of 20 mg./kg. and for that reason the experiment was repeated using 4 doses of 80 mg./kg. which also gave a 100% cure rate.

Example 2

The Effect of Compounds I and II Alone and in Combination Against Chronic Infections of *T. brucei* TREU 667/1

The Compounds I and II which showed promise against 3 day old infections in Example 1 were tested against 21 day old infections of *T. brucei* TREU 667/1 which previous work had shown relapsed after treatment at this time after infection with many of the conventional drugs alone (Berenil, Ethidium, Prothidium, Samorin) even when used at many times the dose rate effective against early infections. Compounds I and II were used singly and in various combinations and the results are shown in Table 2:

TABLE 2

Effectiveness of Compounds I and II Alone and in Combination When Administered to Mice Infected for 21 Days With *Trypanosoma brucei* TREU 667/1

| Drug | Number and Level of Daily Doses | Number Cured* Out of Total Treated |
|---|---|---|
| II | 4 x 20 mg./kg. | 0/9 |
| | 4 x 20 mg./kg. | 0/10 |
| | 4 x 80 mg./kg. | 0/10 |
| II** | 4 x 20 mg./kg. | 0/8 |
| I** | 4 x 20 mg./kg. | 0/9 |
| I* II** | Simultaneously 4 x 20 mg./kg. each | 1/10 |
| I* II** | 4 x 20 mg./kg. II followed by 4 x 20 mg./kg. III | 1/10 |
| II* I** | 4 x 20 mg./kg. III followed by 4 x 20 mg./kg. II | 1/10 |

* Cure indicates that relapses did not occur, i.e., that there was a complete absence of parasites in the blood for at least 90 days after treatment or till the end of the experiment.

** Constituted single experiment.

In every instance the Compounds I and II used, either singly or in various combination, failed to cure the 21 day old infections of *T. brucei* TREU 667/1 in that relapses occurred from 5 days onward.

Example 3

The Effect of Treatment with Other Trypanocidal Drugs Alone and In Combination on Chronic Infections of *T. brucei* TREU 667/1

The effect of pre-treatment on infection of *T. brucei* TREU 667/1 established for 21 days with the three established drugs Ethidium, Prothidium and Samorin is shown in Table 3. Pre-treatment of a 21 day old infection of *T. brucei* TREU 667/1 with Ethidium at 7.5 mg./kg. followed by Compound II at day 24 either in the form of one dose at 80 mg./kg. or 4 doses of 20 mg./kg. each had no effect since the infection subsequently relapsed. The results are set forth in Table 3:

TABLE 3

The Effect of Pretreatment With Ethidium, Prothidium or Samorin on the Efficacy of Compound II on a 21 Day Old Infection of *Trypanosoma brucei* TREU 667/1

| Pretreatment Day 21 | | Treatment Commencing Day 24 | | |
|---|---|---|---|---|
| Drug | Dosage | Drug | Number and Level of Daily Doses | No. Cured* Out of Total Treated |
| Ethidium | 7.5 mg./kg. | — | — | 0/10 |
| Ethidium | 7.5 mg./kg. | II | 4 × 20 mg./kg. | 0/10 |
| Ethidium | 7.5 mg./kg. | II | 1 × 80 mg./kg. | 0/7 |
| Prothidium | 10.0 mg./kg. | — | — | 0/8 |
| Prothidium | 10.0 mg./kg. | II | 4 × 20 mg./kg. | 3/7 |
| Prothidium | 10.0 mg./kg. | II | 1 × 80 mg./kg. | 4/8 |
| Samorin | 2.0 mg./kg. | — | — | 0/10 |
| Samorin | 2.0 mg./kg. | II | 4 × 20 mg./kg. | 0/10 |
| Samorin | 2.0 mg./kg. | II | 1 × 80 mg./kg. | 5/7 |
| — | — | II | 4 × 20 mg./kg. | 0/8 |

* Cure indicates that relapses did not occur, i.e., that there was a complete absence of parasites in the blood for at least 90 days after treatment or till the end of the experiment.

Example 4

The Effect of Treatment with Berenil Alone and In Combination With Compound II on Chronic *T. brucei* TREU 667/1 Infections

The initial work with Berenil was carried out using the dose rate of 40 mg./kg. which was the dose of drug used in all the original work on chronic infections.

TABLE 4

The Effect of Treatment with Berenil Alone and in Combination With Compound II on a 21 Day-old Infection of *Trypanosoma brucei* TREU 667/1

| Pretreatment Day 21 | | Treatment Commencing Day 24 | | |
|---|---|---|---|---|
| Drug | Dose | Drug | Number and Level of Daily Doses | No. Cured* Out of Total Treated |
| Berenil | 5 mg./kg. | — | — | 0/10 |
| Berenil | 5 mg./kg. | Berenil | 5 mg./kg. | 0/10 |
| Berenil | 5 mg./kg. | II | 4 × 20 mg./kg. | 0/9 |
| Berenil | 5 mg./kg. | II | 4 × 80 mg./kg. | 0/10 |
| Berenil | 5 mg./kg. | II | 1 × 20 mg./kg. | 0/10 |
| Berenil | 5 mg./kg. | II | 1 × 80 mg./kg. | 0/10 |
| Berenil | 20 mg./kg. | — | — | 0/10 |
| Berenil | 20 mg./kg. | II<br>I | 4 × 20 mg./kg.<br>each drug | 4/10 |
| Berenil | 40 mg./kg. | — | — | 1/10 |
| Berenil | 40 mg./kg. | — | — | 1/9 |
| Berenil | 40 mg./kg. | II | 4 × 20 mg./kg. | 9/10 |
| Berenil | 40 mg./kg. | II | 1 × 80 mg./kg. | 9/10 |
| Berenil | 40 mg./kg. | II | 4 × 20 mg./kg. | 9/10 |
| Berenil | 40 mg./kg. | — | — | 6/10 |
| Berenil | 40 mg./kg. | II | 4 × 20 mg./kg. | 8/10** |
| Berenil | 40 mg./kg. | II | 1 × 80 mg./kg. | 10/10 |
| Berenil +<br>— | 40 mg./kg. }<br>80 mg./kg. } | — | — | 10/10 |

* Cure indicates that relapses did not occur, i.e., that there was a complete absence of parasites in the blood for at least 90 days after treatment or until the end of the experiment.

** 2 mice died shortly after treatment regimen — no signs of trypanosomes.

In addition, groups of mice were given Berenil and the Compound II simultaneously, or one injection of Berenil followed by one injection of Compound II in order to compress the treatment into the minimum number of injections. The results are set forth in Table 4.

The results showed that if Berenil is administered at 40 mg./kg. followed by Compound II usually administered 3 days after the Berenil either as a single dose (1 × 80 mg.) or 4 doses of 20 mg./kg. then the successful permanent cures were between 90—100%. In one experiment there was an exceptionally high number 6/10 of the mice receiving the Berenil (40 mg./kg.) alone, which were apparently permanently cured, usually this is around 1%.

It was also apparent that pre-treatment with Berenil at lower dose levels was not effective in preventing relapses, although 20 mg./kg. Berenil followed by a combination of the two Compounds II and I each at 20 mg./kg. (4 doses) cured 4/10 mice.

The simultaneous administration of Berenil (40 mg./kg.) and Compound II (80 mg./kg.) also seemed highly effective (100%) until judged against the 6/10 (60%) cure rate for Berenil (40 mg./kg.) given alone in that particular experiment. In all experiments, there seemed little advantage in administering Compound II in 4 doses (4 × 20 mg./kg.) rather than as a single dose (1 × 80 mg./kg.).

Example 5

The Effect of Treatment With Berenil Alone and In Combination With Compound II on Chronic *T. brucei* LUMP 1001 Infections

In order to demonstrate that pre-treatment with Berenil followed by Compound II was effective against different isolates of *T. brucei*, the stabilate *T. brucei* LUMP 1001 was used to establish the infections. It was known that *T. brucei* LUMP 1001 relapsed after treatment of a 21 day old infection Jennings, Whitelaw, Urquart, *Parasitol. 75,* 143—153 (1977), and indeed tended to relapse earlier after treatment with Berenil at 40 mg./kg. than any of the other stabilates tested (approximately 20 days). The original experiment using 40 mg./kg. Berenil as pre-treatment followed by Compound II was disappointing, as only 3 out of a total of 27 mice appeared to be cured by the various dose regimens. The experiment was therefore repeated increasing the pretreatment dose of Berenil to 60 mg./kg. and 80 mg./kg., the latter being given as two doses of 40 mg./kg. on consecutive days to avoid the possibility of deaths due to the toxicity of Berenil at that dose level in mice. This pretreatment was followed by administration of Compound II at 4 doses of 80 mg./kg. and in both instances where the increased Berenil dose level (60 mg./kg. and 80 mg./kg.) was used, satisfactory permanent cures were accomplished, whereas in the respective controls all of the mice relapsed. The results are set forth in Table 5:

TABLE 5

The Effect of Treatment with Berenil Alone and in Combination With Compound II on a 21 Day-old Infection of *Trypanosoma brucei* LUMP 1001

| Pretreatment Day 21 | | Treatment Commencing Day 24 | | |
|---|---|---|---|---|
| Drug | Dose | Drug | Number and Level of Daily Doses | No. Cured* Out of Total Treated |
| Berenil | 40 mg./kg. | — | — | 0/10 |
| Berenil | 40 mg./kg. | II | 4 × 20 mg./kg. | 2/10 |
| Berenil | 40 mg./kg. | II | 1 × 80 mg./kg. | 1/9 |
| ×<br>+ II | 40 mg./kg.<br>80 mg./kg. | — | — | 0/8 |
| Berenil | 40 mg./kg. | — | — | 0/9 |
| Berenil | 60 mg./kg. | — | — | 0/8 |
| Berenil | 80 mg./kg.** | — | — | 0/8 |
| — | — | II | 4 × 80 mg./kg. | 0/10 |
| Berenil | 40 mg./kg. | II | 4 × 80 mg./kg. | 0/8 |
| Berenil | 60 mg./kg. | II | 4 × 80 mg./kg. | 9/12 |
| Berenil | 80 mg./kg. | II | 4 × 80 mg./kg. | 10/10 |

* Cure indicates that relapses did not occur, i.e., that there was a complete absence of parasites in the blood for at least 90 days after treatment or until the end of the experiment.

** Given as 2 × 40 mg./kg. on day 21 and day 24.

The initial results with Compounds I and II tested against early infections (3 day) of *T. brucei* TREU 667/1 showed trypanocidal action in I only at the high level of 4 x 20 mg./kg., a little in Compound II at the low level 4 x 5 mg./kg. and a very definite therapeutic action at the higher level of 4 x 20 mg./kg.

Because of these results, a further group of mice were tested at a higher level (4 x 80 mg./kg.) with Compound I, and the drug at this level cured an early infection.

Experiments using the more promising of the drugs, Compound II, against an established (21 day) infection of *T. brucei* TREU 667/1, showed that even administration of 4 doses at 80 mg./kg. failed to permanently cure the infection, although the last of the mice did not relapse for a period of 42 days after treatment.

Various combinations of Compounds I and II also failed to cure the infection (Table 2).

These results indicate that pre-treatment with Berenil followed by the Compound II, results in a permanent cure in 90—100% of mice which have a long-established infection of *T. brucei,* whereas when Berenil is used alone, usually less than 10% would be free of infection.

It is the combination of the drugs, Berenil plus Compound II which is effective, as neither drug on its own has the same effect. A proportion of mice infected for 21 days with *T. brucei* TREU 667/1 and first treated with Berenil alone (40 mg./kg.) and retreated every 30 days, i.e., before any signs of relapse, for a total of 5 months, have been shown to eventually become parasitaemic again. It is also not entirely due to the concentration of the initial dose of Berenil as 21 day old *T. brucei* TREU 667/1 infections have relapsed after treatment even with 160 mg./kg. Berenil (given subcutaneously); but it is important as it requires a higher level of pre-treatment with Berenil (60 and 80 mg./kg.) in the case of *T. brucei* LUMP 1001 than the *T. brucei* TREU 667/1 to effect a permanent cure. In addition, it would appear that it is not just a question of removal of the mass of trypanosomes circulating in the bloodstream prior to treatment with Compound III, as 5 mg./kg. Berenil will clear all circulating trypanosomes from the bloodstream for up to 20 days (Table 4) and indeed early infections of *T. brucei* TREU 667/1 can be permanently cured at this dose level.

The Compound II could be administered as a single injection rather than in 4 divided doses, and it could also be administered along with the Berenil. This possibility of having a single injection has important practical implications in the treatment of herds of cattle under local African conditions, and would obviate the necessity of continually gathering and treating range cattle.

However, in instances where the dosage of Berenil required was high as against *T. brucei* LUMP 1001, then the effectiveness of dividing the Berenil dose would circumvent the possibility of toxic reactions in the host. In human trypanosomiasis where the consideration of repeated daily treatment is not a major consideration than both the Berenil and Compound II could be given in divided doses. For treatment of trypanosomiasis in humans (Apted, *In "The African Trypanosomiases'* Ed. H. W. Mulligan, George Allen and Unwin, London, 1970.) Berenil is usually given every day for 7—10 days at a dose rate of 2 mg./kg., and indeed can be given orally (Bailey, *Trans. Roy. Soc. Trop. Med. Hyg., 62,* 122, 1968).

The effect of pretreatment with other trypanocidal drugs indicates that some of the mice treated with the prophylactic drugs Prothidium and Samorin did not show signs of relapse until 6—7 months after the treatment, and the combined treatment was more effective than the single pretreatment.

The compositions of this invention have particular value in the control of trypanosomiasis in domesticated animals, particularly cattle. For this purpose, they may be administered in unit dosage forms such as boluses, drenches and preferably by injection. The amount of the composition required for treatment of relapsing trypanosomiasis varies in accordance with such factors as the particular composition employed, the species of animal to be treated, the species of infecting parasite and the severity of infection. In general, the compositions of the present invention comprising Berenil and a substituted nitroimidazole when administered by injection to domestic animals in daily doses of from about 5 mg. to about 100 mg. of Berenil and about 10 mg. to 800 mg. of the nitroimidazole per kilogram of animal body weight are highly effective in treating relapsing trypanosomiasis without intolerable toxic effect. When these compounds are to be employed as therapeutic agents, good results are obtained when the animals are injected with a dose of from about 10 mg. to about 80 mg. of Berenil and about 20 mg. to about 320 mg. of the nitroimidazole and preferably 2 mg. to 50 mg. per kilogram of body weight of Berenil and a dose of 20 mg. to 1·60 mg. of the nitroimidazole compound. Administration of the Berenil and the nitroimidazole may be on the same day or with an interval of about three days. Administration may be in a single dose or divided into a plurality of smaller doses over a period of 24 hours or several days. Where prophylactic treatment is desired and the compounds are administered continuously, satisfactory results are obtained when the animals ingest daily dosages of about 0.1 mg. to 100 mg. of the composition per kilogram of body weight. The unit dosage forms may be readily prepared by conventional formulating techniques and are particularly useful when administration is to be made in a single dose or divided doses over a period of 24 hours.

The exact amount of active ingredient to be employed in the above compositions may vary provided that a sufficient amount is ingested to give the required dosage. In general, tablets, boluses, drenches and intraperitoneal injections containing from about 5 to 70 percent weight of active ingredient may be satisfactorily employed to supply the desired dosage.

When Berenil and the substituted nitroimidazoles of this invention are used in the prevention and

treatment of human "sleeping sickness", the compounds can be administered as intravenous, or intraperitoneal injections. The compounds are suspended or dissolved in an inert non-toxic pharmaceutically acceptable carrier and administered. When used as a prophylactic, 2—12 mg./kg. of body weight are injected every 1—8 months. Certain of the nitroimidazole compounds can also be used orally or intraperitoneally against Chagas' disease which is caused by *Trypanosoma cruzi.* The oral dosage is 50—250 mg. per kilogram twice a day for 5—10 days.

When the novel compositions of the present invention are used therapeutically to treat well established infections of trypanosomiasis, a preferred method of treatment is to administer Berenil at a dose range of about 2 mg. to about 50 mg. per kilogram of body weight and 20 mg. to 160 mg. per kilogram of body weight of the nitroimidazole administered concomitantly.

**Claims**

1. A composition suitable for the treatment of chronic trypanosomiasis infections comprising diminazene aceturate and a nitroimidazole having the structure

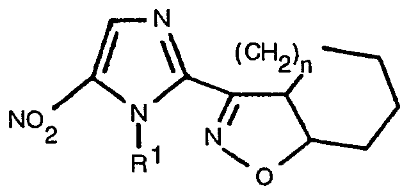

wherein $R^1$ is hydrogen or $C_{1-6}$ alkyl, and *n* is 1 or 3 in a weight ratio of 1—8:2—32.

2. A composition as claimed in Claim 1 in which in the formula $R^1$ is methyl and *n* is 1.

3. A composition as claimed in Claim 1 in which in the formula $R^1$ is methyl and *n* is 3.

4. A mixture of compounds as claimed in Claim 1, for use in the treatment of chronic trypanosomiasis infections in humans and animals.

**Revendications**

1. Composition appropriée au traitement des infections de trypanosomiase chronique, comprenant de l'acéturate de diminazène et un nitroimidazole de structure

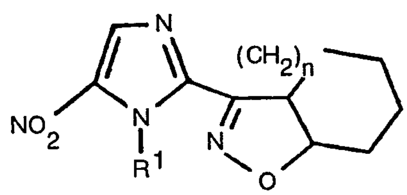

où $R^1$ est un hydrogène ou un alcoyle en $C_1$ à $C_6$, et où *n* vaut 1 ou 3, dans un rapport pondéral de 1—8:2—32.

2. Composition selon la revendication 1 où dans la formule $R^1$ est un méthyle et *n* vaut 1.

3. Composition selon la revendication 1 où dans la formule $R^1$ est un méthyle et *n* vaut 3.

4. Mélange de composés selon la revendication 1, aux fins d'application dans le traitement des infections de trypanosomiase chronique chez l'homme et l'animal.

**Patentansprüche**

1. Zusammensetzung geeignet zur Behandlung von chronischen Trypanosomiasisinfektionen, enthaltend Diminazen-Aceturat und ein Nitroimidazol mit der Struktur

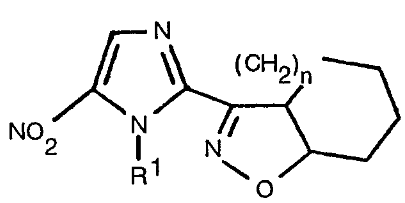

worin $R^1$ Wasserstoff oder $C_{1-6}$-Alkyl ist und n 1 oder 3 ist, in einem Gewichtsverhältnis von 1—8:2—32.

2. Zusammensetzung nach Anspruch 1, in der in der Formel $R^1$ Methyl ist und n 1 ist.

3. Zusammensetzung nach Anspruch 1, in der in der Formel $R^1$ Methyl ist und n 3 ist.

4. Ein Gemisch von Verbindungen nach Anspruch 1, zur Verwendung bei der Behandlung von chronischen Trypanosomiasisinfektion von Menschen und Tieren.